(19) Europäisches Patentamt
European Patent Office
Office européen des brevets

(11) **EP 4 016 379 B1**

(12) **EUROPEAN PATENT SPECIFICATION**

(45) Date of publication and mention
of the grant of the patent:
**31.01.2024 Bulletin 2024/05**

(21) Application number: **20918621.2**

(22) Date of filing: **09.12.2020**

(51) International Patent Classification (IPC):
**H01J 49/00** (2006.01)    **G06F 18/00** (2023.01)

(52) Cooperative Patent Classification (CPC):
**H01J 49/0036; G06F 2218/06; G06F 2218/10**

(86) International application number:
**PCT/CN2020/134810**

(87) International publication number:
**WO 2021/159833 (19.08.2021 Gazette 2021/33)**

(54) **NUCLEIC ACID MASS SPECTRUM NUMERICAL PROCESSING METHOD**

VERFAHREN ZUR NUMERISCHEN VERARBEITUNG VON NUKLEINSÄUREMASSENSPEKTREN

PROCÉDÉ DE TRAITEMENT NUMÉRIQUE DE SPECTRE DE MASSE D'ACIDE NUCLÉIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.02.2020 CN 202010084107**

(43) Date of publication of application:
**22.06.2022 Bulletin 2022/25**

(73) Proprietor: **Zhejiang Digena Diagnostic Technology Co., Ltd.**
**Hangzhou, Zhejiang 311100 (CN)**

(72) Inventors:
• **SHU, Jianwei**
**Hangzhou, Zhejiang 311100 (CN)**
• **XIANG, Shuanghong**
**Hangzhou, Zhejiang 311100 (CN)**
• **WANG, Songjiong**
**Hangzhou, Zhejiang 311100 (CN)**

(74) Representative: **Gulde & Partner**
**Patent- und Rechtsanwaltskanzlei mbB**
**Wallstraße 58/59**
**10179 Berlin (DE)**

(56) References cited:
**EP-A1- 1 469 313      CN-A- 110 196 274**
**CN-A- 110 441 253      CN-A- 111 325 121**

**US-A1- 2003 180 748      US-A1- 2014 306 104**
**US-A1- 2016 240 359      US-A1- 2019 172 694**

• **Anonymous: "High Definition Imaging (HDI) 1.4: Lock Mass Recalibration for MS Imaging Data Sets", , 15 November 2016 (2016-11-15), XP055977787, Retrieved from the Internet: URL:https://www.waters.com/webassets/cms/library/docs/720005809en.pdf [retrieved on 2022-11-04]**
• **P. DU ET AL: "Improved peak detection in mass spectrum by incorporating continuous wavelet transform-based pattern matching", BIOINFORMATICS, vol. 22, no. 17, 4 July 2006 (2006-07-04), pages 2059-2065, XP055259624, GB ISSN: 1367-4803, DOI: 10.1093/bioinformatics/btl355**
• **FRANK-MICHAEL SCHLEIF ET AL: "Support vector classification of proteomic profile spectra based on feature extraction with the bi-orthogonal discrete wavelet transform", COMPUTING AND VISUALIZATION IN SCIENCE, SPRINGER, BERLIN, DE, vol. 12, no. 4, 7 March 2008 (2008-03-07), pages 189-199, XP019724063, ISSN: 1433-0369**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- Carroll James A. ET AL: "Using Matrix Convolution Filters to Extract Information from Time-of-flight Mass Spectra", Rapid Communications in Mass Spectrometry, vol. 10, no. 13, 1 October 1996 (1996-10-01), pages 1683-1687, XP093075854, GB ISSN: 0951-4198, DOI: 10.1002/(SICI)1097-0231(199610)10:13<1683: :AI D-RCM716>3.0.CO;2-L

**Description**

TECHNICAL FIELD

The present disclosure belongs to a technical field of nucleic acid mass spectrum, and particularly relates to a numerical processing method for a nucleic acid mass spectrum.

BACKGROUND

Mass spectrum technology has been widely used in biological analysis in recent years because of the advantages of rapidity, accuracy, and high sensitivity. As the basic material of life, nucleic acid plays a vital role in the maj or life phenomena, such as growth, development, reproduction, inheritance, and variation, of organisms. Modern biotechnology has found that most of the physiological or disease traits are expressed by a series of gene regulation existing in the nucleic acid sequence. Therefore, for the nucleic acid, accurate nucleotide detection is particularly important. As an indispensable part before the nucleotide detection, numerical processing of a mass spectrum is of self-evident importance. At present, there are problems such as low conversion rate of the acquired data and uneven data in similar methods, these problems seriously affect the result of the nucleotide detection, and the further research is needed in this regard.

US 2014/306104 A1 describes a high-throughput mass-spectrometric characterization of samples. The invention relates to the characterization of samples which are located in their many hundreds up to tens or hundreds of thousands on a sample support plate in a regular pattern, a so-called array, by ionization with matrix-assisted laser desorption and mass spectrometric measurement. US 22016/240359 A1 describes a dynamic resolution correction of a quadrupole mass analyzer. EP1469313A1 describes a method of mass spectrometry. US 2003/180748 A1 describes methods for generating databases for identifying polymorphic genetic markers.

SUMMARY

The purpose of the present disclosure is to solve the problems of low conversion rate, uneven data, and the like in the mass spectrum data acquisition process, and the present disclosure provides a numerical processing method for a nucleic acid mass spectrum to extract reliable feature values before gene analysis, and the numerical processing method is a numerical processing method for a nucleic acid mass spectrum aiming at ameliorating the limitation of the prior art and improving the accuracy of nucleotide detection.

It is an object of the present invention to provide a numerical processing method for a nucleic acid mass spectrum. The object is achieved by the features of the respective independent claims. Further embodiments are defined in the respective dependent claims.

The present disclosure has the following advantages: 1. the numerical processing method for the nucleic acid mass spectrum provided by the present disclosure can extract reliable feature values before the gene analysis, and is a numerical processing method for a nucleic acid mass spectrum aiming at ameliorating the limitation of the prior art and improving the accuracy of nucleotide detection; 2. the numerical processing method for the nucleic acid mass spectrum can improve the credibility of nucleic acid mass spectrum data acquisition.

BRIEF DESCRIPTION OF THE DRAWINGS

FIG. 1: mass spectrum before filtering;
FIG. 2: mass spectrum after filtering;
FIG. 3: comparison diagram before and after peak fitting.

DETAILED DESCRIPTION

The content of the present disclosure is further described below with reference to the accompanying drawing and embodiments.

The present disclosure relates to a numerical processing method for a nucleic acid mass spectrum, and the numerical processing method comprises the following steps:

step S1: recalibrating a single mass spectrum. For a sample, a plurality of mass spectra (e.g., n mass spectra, and typically n = 5) corresponding to different positions of a detection point of the sample. Each mass spectrum is actually a sum of the mass spectra obtained by a plurality of times of laser excitation (e.g., m times, and typically m = 20). An initial coefficient of the mass spectrum is generated based on the assumption that the mapping function between the mass spectrometer and m/z (mass-to-charge ratio) is a quadratic function (a form of the function is: $m=At^2+Bt+C$). Before summing the mass spectra, the recalibration of the mass spectra is needed. The process of the recalibration is accomplished by matching a group of special identified peaks (called anchor peaks) to the expected mass thereof and follows the following steps:

In step S11: selecting a candidate reference peak, and selecting a group of clean reference peaks from all possible expected peaks according to the following criteria:

1. A peak value of the reference peak must be within a mass range of 4000 Da to 9000 Da.
2. The reference peak has no adjacent reference peak within the mass range defined by the mass +/- resolution.

In step S12, positioning a peak, and applying a weight matrix convolution filter with a width of 9 to the mass spectrum. The matrix is preferably: (-4, 0, 1, 2, 2, 2, 1, 0, -4). For a given point of the mass spectrum, an intensity value of the given point after applying the weight matrix convolution filter is equal to a weighted sum of 9 values around the given point, and is expressed by a following formula:

$$y_i' = \sum_{k=1}^{9} I_k y_k + i$$

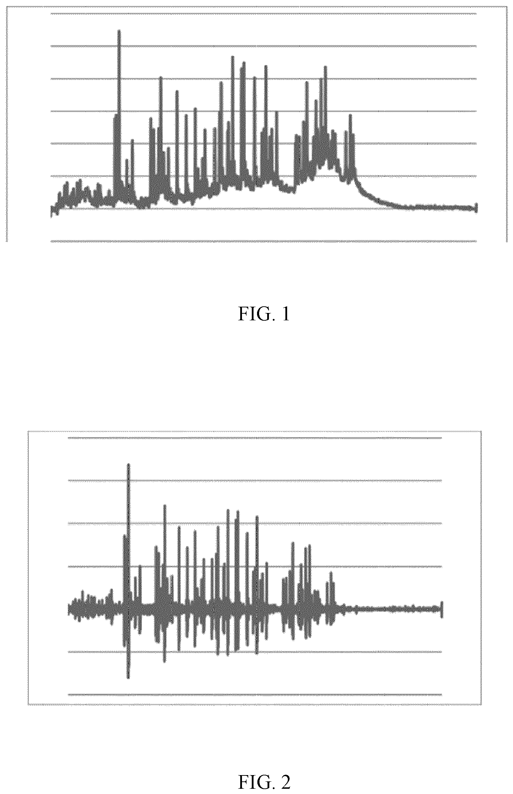

Based on the filtered intensity values, a small sliding window (n=+/-3) is used to identify a local maximum. Then, the whole mass spectrum is divided into a plurality of intervals and each interval has 500 points. For each interval, the local noise is identified as 33% of the local maximum within the surrounding window having 1500 points (+/- an interval). A peak with an intensity greater than or equal to four times the local noise and greater than or equal to a global minimum value is identified as a candidate peak, the global minimum value is preferably 0.01 times a maximum local maximum value (namely, 0.01 * the maximum local maximum value). For an identified peak list, a peak is removed, where a reference peak adjacent to the peak exists within a certain range, and the peak has a SNR (ratio of the filtered intensity value to the local noise) ≤ 2 and has a mass value outside the range of the pre-specified candidate reference peaks. Finally, the peak value index is adjusted based on the original intensity. The mass spectrum before and after the application of the filter may be referred to the FIG. 1 and FIG.2.

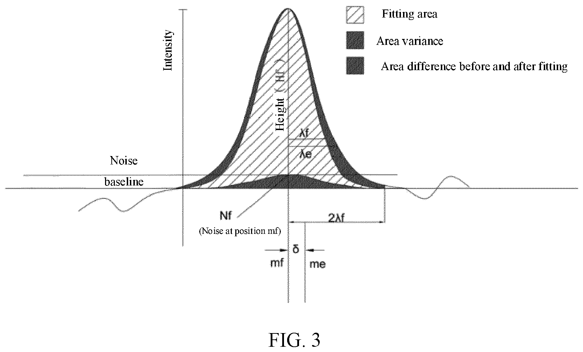

In the step S13, fitting a peak of the mass spectrum, as shown in FIG. 3, the specific implementation steps are as follows:

Step S131: determining an expected line width. The expected line width is determined by using the following formula: $\lambda_e = L_A + L_B \cdot M$, where $L_A$ and $L_B$ are the default parameters (the default value of $L_A$ is 2.5, and the default value of $L_B$ is 0.0005), and M is the given peak value (Da).

Step S132: masking a region of an expected signal within an interval of NN expected line widths, NN preferably being 4.

Step S133: calculating an average of an intensity $y_i$ of the mass spectrum within a $MM\lambda_m$ interval as an implicit baseline, where $\lambda_m$ is the smallest estimated line width in this $MM\lambda_m$ interval, and MM is preferably 80, and in the masked region of this $MM\lambda_m$ interval, the intensity yi is provided by linear interpolation.

Step S134: calculating the effective value (Root Mean Square,RMS) of the (signal-baseline) operation as the noise level.

Step S135: further masking a point, having a SNR (SNR calculated as the ratio of the peak height to the noise) great than 5 and a noise greater than 1, in the peak region.

Step S136: determining a region within four estimated line widths of each peak as a fitted region, and in a case of no overlapping peaks, fitting a single Gaussian peak by Levenberg-Marquardt algorithm to find parameters $M_f$ (fitting mass) and $\lambda_f$ (fitting line width), so that the tuning function (prototype of the function is shown below) is minimized.

$$\chi^2 = \Sigma \left[ \frac{y_i - H_f \cdot \exp\left[ -\left( \frac{M_f - m_i)^2}{\lambda_f} \right) \right]}{\sigma_i} \right]^2$$

The sum is obtained by summing all $\{y_i, m_i\}$ from the specified interval, $H_f$ is the fitting height above the baseline corresponding to the point $M_f$. For a point in the region with a distance from the peak center within $0.5\lambda_e$, $\sigma_i$ of the point is set equal to 1, and for a point in the region with a distance from the peak center beyond $0.5\lambda_e$, $\sigma_i$ of the point is set to 0.2 or 0.4.

Step S14: finally selecting an anchor peak, for a detected peak list, finding a cut-off SNR (i. e., a minimum SNR), matching a peak in the detected peak list with a list of candidate reference peaks, and only selecting a peak whose mass is within a +/- 25Da of the candidate reference peak and whose SNR is higher than the cut-off SNR.

Step S15: performing a recalibration, calculating a calibration coefficient by a nonlinear fitting method in combination with the anchor peak obtained and an expected mass of the anchor peak. Here, it is assumed that a mapping function between a mass spectrometer and m/z (mass-to-charge ratio) is a Bruker function in a form of

$$m = A(\sqrt{Bt + C} - 1)^2.$$

In the step S2, synthesizing the plurality of mass spectra, that is to say, on a basis of the step S1, the plurality of mass spectra corresponding to different positions of the detection point are synthesized into one mass spectrum of the detection point. The method of synthesizing the plurality of mass spectra is a "self-weighted average" method that can be described by using the following equation:

$$\overline{I}_i = \frac{\sum_{j=1}^{n} I_{ij}^{2}}{\sum_{j=1}^{n} |I_{ij}|}$$

where n is a count of the plurality of mass spectra, $\overline{I}_i$ is the average intensity of mass i; $I_{ij}$ is an intensity of the mass i from a j-th mass spectrum.

When the mass spectra have different calibration coefficients, the optimal mass spectrum with the most anchor peaks is selected from the mass spectra. The summed mass spectrum (that is, the mass spectrum obtained by performing the "self-weighted average" method on the plurality of mass spectra) is initialized with the optimal mass spectrum. Only when the calibration coefficient of a mass spectrum and the calibration coefficient of the optimal mass spectrum meet the condition (A should change within 1%; B should change within 10%; C should change within 20Da), the absolute intensity or the square intensity of the mass spectrum can be summed with the absolute intensity or the square intensity of another mass spectrum.

Step S3, performing wavelet filtering, the wavelet filtering being performed on the synthesized mass spectrum to eliminate the high-frequency noise and the baseline, and then, performing another round of recalibration on the filtered mass spectrum. After this round of recalibration, assigning a new coefficient A, a new coefficient B, a new coefficient C to the synthesized mass spectrum and adjusting the m/z (mass-to-charge ratio) value accordingly.

Step S4, extracting a peak feature value, as shown in FIG. 3, and the fitting process follows the following steps:

step S41: fitting a peak, the same as the step S13;
step S42: recording following features after the fitting is successful:

    1. a height above a baseline of a center of a fitted peak, $H_f$,
    2. a fitting line width $\lambda_f$,
    3. a peak offset ( a distance between the center of the fitted peak and a center of an expected peak), $\delta_f = M_f - M_e$,
    4. an area A of a region between the fitted peak and the baseline within a range of $4\lambda_f$,
    5. a signal-noise ratio, $SNR = H_f / N(M_f)$,
    6. an area variance, $V = A/SNR$,
    7. a fitting area difference $\Delta$, a square root of a sum of a square difference between a fitted intensity and a measured intensity

The numerical processing method for the nucleic acid mass spectrum according to the present disclosure can extract the reliable feature values before gene analysis, and is a numerical processing method for a nucleic acid mass spectrum aiming at ameliorating the limitation of the prior art and improving the accuracy of nucleotide detection; in addition, the method can improve the reliability of the acquired nucleic acid mass spectrum data.

**Claims**

1. A numerical processing method for a nucleic acid mass spectrum, comprising:

step S1: recalibrating mass spectra, comprising for each detection point of a sample, obtaining a plurality of mass spectra corresponding to different positions of the detection point, wherein each single mass spectrum of the plurality of mass spectra is recalibrated by using a group of anchor peaks with an expected mass-to-charge ratio;

step S2, synthesizing the plurality of mass spectra, wherein on a basis of the step S1, the plurality of mass spectra corresponding to the different positions of the detection point are synthesized into a unitary mass spectrum of the detection point;

step S3: performing wavelet filtering on the unitary mass spectrum of the detection point, on a basis of the step S2, to eliminate high-frequency noise and a baseline through a wavelet-based digital filter; and

step S4: extracting a peak feature value, performing peak fitting to obtain a fitted curve of the unitary mass spectrum of the detection point on a basis of the step S3, and obtaining a peak height, a peak width, a peak area, a mass offset, and a signal-noise ratio based on the fitted curve of the unitary mass spectrum of the detection point,

wherein in the step S1, recalibrating each single mass spectrum comprises:

step S11: selecting a group of reference peaks, wherein selecting a group of reference peaks comprises selecting the group of reference peaks from all possible expected peaks according to following criteria:

(1) a peak value of the reference peak being within a mass range of a specific interval, the mass range of the specific interval being from 4000 Da to 9000 Da;

(2) the reference peak having no adjacent reference peak in a mass range defined by the mass of the reference peak +/- resolution;

step S12, positioning a peak, and applying a weight matrix convolution filter with a width of 9 to the single mass spectrum, wherein the weight matrix convolution filter is: (-4, 0, 1, 2, 2, 2, 1, 0, -4), for a given point of the single mass spectrum, an intensity value of the given point after applying the weight matrix convolution filter is equal to a weighted sum of 9 values around the given point, and is expressed by a following formula:

$$y'_i = \sum_{k=1}^{9} I_k y_k + i$$

decomposing the single mass spectrum into a plurality of specific point intervals based on filtered intensity values, identifying a local noise for each specific point interval, and identifying a peak with an intensity greater than or equal to four times the local noise and greater than or equal to a global minimum value as a reference peak, wherein the global minimum value is: 0.01 * a maximum local maximum value;

step S13: fitting a peak of the single mass spectrum;

step S14: finally selecting an anchor peak, for a detected peak list, finding a cut-off signal-noise ratio, matching a peak in the detected peak list with a list of the reference peaks, and only selecting a peak whose mass is within a specific range of the reference peak and whose signal-noise ratio is higher than the cut-off signal-noise ratio;

step S15: performing a recalibration operation, calculating a calibration coefficient by a nonlinear fitting method in combination with the anchor peak and an expected mass of the anchor peak, wherein a mapping function between a mass spectrometer and mass-to-charge ratio is a Bruker function in a form of

$$m = A(\sqrt{Bt + C} - 1)^2.$$

2. The numerical processing method for the nucleic acid mass spectrum according to claim 1, wherein the step S2 comprises:

synthesizing the plurality of mass spectra by using a self-weighted average method,

selecting an optimal mass spectrum with the most anchor peaks from the plurality of mass spectra in a case where the plurality of mass spectra have different calibration coefficients;

initializing a mass spectrum synthesized by performing the self-weighted average method on the plurality of mass spectra with the optimal mass spectrum;

summing an absolute intensity or a square intensity of a mass spectrum with an absolute intensity or a square intensity of another mass spectrum only in a case where a calibration coefficient of the mass spectrum and a calibration coefficient of the optimal spectrum meet a condition, wherein the condition is A changes within 1%, B changes within 10%, and C changes within 20 Da.

3. The numerical processing method for the nucleic acid mass spectrum according to claim 1, wherein the step S3 comprises:

performing wavelet filtering on the unitary mass spectrum of the detection point to eliminate the high-frequency noise and the baseline to obtain a filtered mass spectrum, and then performing another round of recalibration on the filtered mass spectrum and adjusting a mass-to-charge ratio value accordingly.

4. The numerical processing method for the nucleic acid mass spectrum according to claim 1, wherein the step S4 comprises:

step S41: fitting a peak of the unitary mass spectrum to obtain a fitted peak;

step S42: recording following features:

(1) a height above a baseline of a center of the fitted peak, $H_f$,
(2) a fitting line width, $\lambda_f$,
(3) a peak offset, which is a distance between the center of the fitted peak and a center of an expected peak, $\delta_f = M_f - M_e$,
(4) an area A of a region between the fitted peak and the baseline within a range of $4\lambda_f$,
(5) a signal-noise ratio, $SNR = H_f/N(M_f)$,
(6) an area variance, $V = A/SNR$,
(7) a fitting area difference $\Delta$, a square root of a sum of a square difference between a fitted intensity and a measured intensity.

5. The numerical processing method for the nucleic acid mass spectrum according to claim 1, wherein in the step S13, steps of fitting a peak comprises:

step S13 1: determining an expected line width;
step S132: masking a region of an expected signal within an interval of NN expected line widths, NN being 4;
step S133: calculating an average of an intensity yi of the single mass spectrum within a MM$\lambda$m interval as an implicit baseline, wherein $\lambda$m is a smallest estimated line width in the MM$\lambda$m interval, MM is 80, and in a masked region of the MM$\lambda$m interval, a value of the intensity yi is provided by linear interpolation;
step S134: calculating an effective value of a signal-baseline operation as a noise level;
step S135: masking a point, and further masking a point, having a signal-noise ratio, which is calculated as a ratio of the peak height to a noise, greater than a ratio given value and a noise greater than a noise given value, in a peak region;
step S136, determining a region having specific estimated line widths of each peak as a fitted region, and in a case of no overlapping peaks, fitting a single Gaussian peak by Levenberg-Marquardt algorithm to find specified parameters to minimize a tuning function.

6. The numerical processing method for the nucleic acid mass spectrum according to claim 2, wherein the self-weighted average method is described by using a following equation:

$$\overline{I}_i = \frac{\sum_{j=1}^{n} I_{ij}^{\ 2}}{\sum_{j=1}^{n} \left| I_{ij} \right|},$$

wherein n is a count of the plurality of mass spectra, $\bar{I}_i$ is an average intensity of mass i; $I_{ij}$ is an intensity of the mass i from a j-th mass spectrum.

7. The numerical processing method for the nucleic acid mass spectrum according to claim 5, wherein in the step S131, the expected line width determined is described by a following equation:

$$\lambda_e \ = \ L_A \ + \ L_B \ \cdot \ M$$

wherein $L_A$ and $L_B$ are default parameters, and M is a given peak value.

8. The numerical processing method for the nucleic acid mass spectrum according to claim 5, wherein the tuning function in the step S136 is described by a following equation:

$$\chi^2 \ = \ \Sigma \left[ \frac{y_i \ - \ H_f \ \bullet \ \exp\left[ - (\frac{(M_f \ - \ m_i)^2}{\lambda_f}) \right]}{\sigma_i} \right]^2$$

wherein a sum is obtained by summing all $\{y_i, m_i\}$ from a specified interval, $H_f$ is a fitting height above the baseline corresponding to a point $M_f$, a parameter $M_f$ represents a fitting mass, a parameter $\lambda_f$ represents a fitting line width, and $\sigma_i$ is a certain parameter according to a given condition.

**Patentansprüche**

1. Verfahren zur numerischen Verarbeitung eines Nukleinsäuremassenspektrums, umfassend:

Schritt S1: Rekalibrieren von Massenspektren, umfassend für jeden Erfassungspunkt einer Probe das Erhalten einer Vielzahl von Massenspektren, die verschiedenen Positionen des Erfassungspunkts entsprechen, wobei jedes einzelne Massenspektrum der Vielzahl von Massenspektren unter Verwendung einer Gruppe von Ankerpeaks mit einem erwarteten Masse-Ladungs-Verhältnis rekalibriert wird;
Schritt S2, Synthetisieren der Vielzahl von Massenspektren, wobei auf der Grundlage des Schritts S1 die Vielzahl von Massenspektren, die den verschiedenen Positionen des Erfassungspunkts entsprechen, in ein einheitliches Massenspektrum des Erfassungspunkts synthetisiert werden;
Schritt S3: Durchführen einer Wavelet-Filterung des einheitlichen Massenspektrums des Erfassungspunkts auf der Grundlage des Schritts S2, um hochfrequentes Rauschen und eine Grundlinie zu eliminieren, durch einen digitalen Filter auf Wavelet-Basis; und
Schritt S4: Extrahieren eines Peak-Merkmalswertes, Durchführen einer Peak-Anpassung, um eine angepasste Kurve des einheitlichen Massenspektrums des Erfassungspunktes auf der Grundlage des Schrittes S3 zu erhalten, und Erhalten einer Peak-Höhe, einer Peak-Breite, einer Peak-Fläche, eines Massen-Offsets und eines Signal-Rausch-Verhältnisses auf der Grundlage der angepassten Kurve des einheitlichen Massenspektrums des Erfassungspunktes,

wobei in dem Schritt S1 das Rekalibrieren jedes einzelnen Massenspektrums umfasst:

Schritt S11: Auswählen einer Gruppe von Referenzpeaks, wobei das Auswählen einer Gruppe von Referenzpeaks das Auswählen der Gruppe von Referenzpeaks aus allen möglichen erwarteten Peaks gemäß den folgenden Kriterien umfasst:

(1) ein Peakwert des Referenzpeaks liegt innerhalb eines Massenbereichs eines spezifischen Intervalls, wobei der Massenbereich des spezifischen Intervalls von 4000 Da bis 9000 Da reicht;
(2) der Referenzpeak hat keinen benachbarten Referenzpeak in einem Massenbereich, der durch die Masse des Referenzpeaks +/- Auflösung definiert ist;

Schritt S12, Positionieren eines Peaks und Anwenden eines Gewichtungsmatrix-Faltungsfilters mit einer Breite von 9 auf das einzelne Massenspektrum, wobei der Gewichtungsmatrix-Faltungsfilter ist: (-4, 0, 1, 2, 2, 2, 1, 0, -4) ist, wobei für einen gegebenen Punkt des einzelnen Massenspektrums ein Intensitätswert des gegebenen Punktes nach Anwendung des Gewichtungsmatrix-Faltungsfilters gleich einer gewichteten Summe von 9 Werten um den gegebenen Punkt ist und durch eine folgende Formel ausgedrückt wird:

$$y'_i = \sum_{k=1}^{9} I_k y_k + i$$

Zerlegen des einzelnen Massenspektrums in eine Vielzahl von spezifischen Punktintervallen auf der Grundlage von gefilterten Intensitätswerten, Identifizieren eines lokalen Rauschens für jedes spezifische Punktintervall und Identifizieren eines Peaks mit einer Intensität, die größer oder gleich dem Vierfachen des lokalen Rauschens und größer oder gleich einem globalen Minimalwert ist, als einen Referenzpeak, wobei der globale Minimalwert 0,01 * ein maximaler lokaler Maximalwert ist;

Schritt S13: Anpassen eines Peaks des einzelnen Massenspektrums;

Schritt S14: schließlich Auswählen eines Anker-Peaks für eine Liste der erfassten Peaks, Ermitteln eines Abschaltsignal-Rausch-Verhältnisses, Abgleichen eines Peaks in der Liste der erfassten Peaks mit einer Liste der Referenzpeaks und Auswählen nur eines Peaks, dessen Masse innerhalb eines bestimmten Bereichs des Referenzpeaks liegt und dessen Signal-Rausch-Verhältnis höher ist als das Abschaltsignal-Rausch-Verhältnis;

Schritt S15: Durchführen einer Rekalibrierungsoperation, Berechnen eines Kalibrierungskoeffizienten durch ein nichtlineares Anpassungsverfahren in Kombination mit dem Ankerpeak und einer erwarteten Masse des Ankerpeaks, wobei eine Abbildungsfunktion zwischen einem Massenspektrometer und einem Masse-Ladungs-Verhältnis eine Bruker-Funktion in der Form $m = A(\sqrt{Bt + C} - 1)^2$ ist.

2. Verfahren zur numerischen Verarbeitung des Nukleinsäuremassenspektrums nach Anspruch 1, wobei der Schritt S2 umfasst:

Synthetisieren der Vielzahl von Massenspektren unter Verwendung eines Verfahrens des selbstgewichteten Durchschnitts,

Auswählen eines optimalen Massenspektrums mit den meisten Ankerpeaks aus der Vielzahl von Massenspektren in einem Fall, in dem die Vielzahl von Massenspektren unterschiedliche Kalibrierkoeffizienten aufweist;

Initialisieren eines Massenspektrums, das durch Ausführen des Verfahrens des selbstgewichteten Durchschnitts an der Vielzahl von Massenspektren synthetisiert wurde, mit dem optimalen Massenspektrum;

Summieren einer absoluten Intensität oder einer quadratischen Intensität eines Massenspektrums mit einer absoluten Intensität oder einer quadratischen Intensität eines anderen Massenspektrums nur in einem Fall, in dem ein Kalibrierungskoeffizient des Massenspektrums und ein Kalibrierungskoeffizient des optimalen Spektrums eine Bedingung erfüllen, wobei die Bedingung ist, dass A sich innerhalb von 1% ändert, B sich innerhalb von 10% ändert und C sich innerhalb von 20 Da ändert.

3. Verfahren zur numerischen Verarbeitung des Nukleinsäuremassenspektrums nach Anspruch 1, wobei der Schritt S3 umfasst:

Durchführen einer Wavelet-Filterung des einheitlichen Massenspektrums des Erfassungspunktes, um das hochfrequente Rauschen und die Grundlinie zu eliminieren, um ein gefiltertes Massenspektrum zu erhalten, und anschließendes Durchführen einer weiteren Runde der Rekalibrierung des gefilterten Massenspektrums und entsprechendes Einstellen eines Masse-Ladungs-Verhältniswertes.

4. Verfahren zur numerischen Verarbeitung des Nukleinsäuremassenspektrums nach Anspruch 1, wobei der Schritt S4 umfasst:

Schritt S41: Anpassen eines Peaks des einheitlichen Massenspektrums, um einen angepassten Peak zu erhalten;

Schritt S42: Aufzeichnen der folgenden Merkmale:

(1) eine Höhe über der Grundlinie des Mittelpunkts des angepassten Peaks, $H_f$,

(2) eine Breite der Anpassungslinie, $\lambda_f$,

(3) ein Peak-Offset, d. h. ein Abstand zwischen dem Zentrum des angepassten Peaks und dem Zentrum eines erwarteten Peaks, $\delta_f = M_f - M_e$,

(4) eine Fläche A eines Bereichs zwischen dem angepassten Peak und der Grundlinie innerhalb eines Bereichs von $4\lambda_f$,

(5) ein Signal-Rausch-Verhältnis, $SNR = H_f/N(M)_f$,

(6) eine Flächenvarianz, $V = A/SNR$,

(7) eine Anpassungsflächendifferenz $\Delta$, die Quadratwurzel aus der Summe der quadratischen Differenz zwischen einer angepassten Intensität und einer gemessenen Intensität.

5. Verfahren zur numerischen Verarbeitung des Nukleinsäuremassenspektrums Anspruch 1, wobei im Schritt S13 die Schritte der Anpassung eines Peaks umfassen:

Schritt S131: Bestimmen einer erwarteten Linienbreite;

Schritt S132: Maskieren eines Bereichs eines erwarteten Signals innerhalb eines Intervalls von NN erwarteten Linienbreiten, wobei NN 4 ist;

Schritt S133: Berechnen eines Durchschnitts einer Intensität yi des einzelnen Massenspektrums innerhalb eines MM$\lambda$m-Intervalls als implizite Grundlinie, wobei $\lambda$m eine kleinste geschätzte Linienbreite in dem MM$\lambda$m-Intervall ist, MM gleich 80 ist und in einem maskierten Bereich des MM$\lambda$m-Intervalls ein Wert der Intensität yi durch lineare Interpolation bereitgestellt wird;

Schritt S134: Berechnen eines Effektivwerts einer Signal-Grundlinien-Operation als Rauschpegel;

Schritt S135: Maskieren eines Punktes und weiteres Maskieren eines Punktes mit einem Signal-Rausch-Verhältnis, das als Verhältnis der Peakhöhe zu einem Rauschen berechnet wird, das größer ist als ein gegebener Verhältniswert und ein Rauschen, das größer ist als ein gegebener Rauschwert, in einem Peakbereich;

Schritt S136: Bestimmen eines Bereichs mit spezifischen geschätzten Linienbreiten jedes Peaks als angepasster Bereich und, falls es keine überlappenden Peaks gibt, Anpassen eines einzelnen Gauß-Peaks durch den Levenberg-Marquardt-Algorithmus, um bestimmte Parameter zur Minimierung einer Abstimmungsfunktion zu finden.

6. Verfahren zur numerischen Verarbeitung des Nukleinsäuremassenspektrums nach Anspruch 2, wobei das Verfahren des selbstgewichteten Durchschnitts durch Verwendung der folgenden Gleichung beschrieben wird:

$$\overline{I_i} = \frac{\sum_{j=1}^{n} I_{ij}^2}{\sum_{j=1}^{n} |I_{ij}|},$$

wobei n eine Anzahl der Vielzahl von Massenspektren ist, $\overline{I_i}$ eine durchschnittliche Intensität der Masse i ist; $I_{ij}$ eine Intensität der Masse i aus einem j-ten Massenspektrum ist.

7. Verfahren zur numerischen Verarbeitung des Nukleinsäuremassenspektrums nach Anspruch 5, wobei im Schritt S131 die ermittelte erwartete Linienbreite durch die folgende Gleichung beschrieben wird:

$$\lambda_e = L_A + L_B \cdot M$$

wobei $L_A$ und $L_B$ Standardparameter sind und M ein bestimmter Peak-Wert ist.

8. Verfahren zur numerischen Verarbeitung des Nukleinsäuremassenspektrums nach Anspruch 5, wobei die Abstimmungsfunktion im Schritt S136 durch die folgende Gleichung beschrieben wird:

$$\chi^2 = \Sigma \left[ \frac{y_i - H_f \bullet \exp\left[ - (\frac{M_f - m_i}{\lambda_f})^2 \right]}{\sigma_i} \right]^2$$

wobei eine Summe durch Aufsummieren aller $\{y_i, m_i\}$ aus einem bestimmten Intervall erhalten wird, $H_f$ eine Anpassungshöhe über der Grundlinie ist, die einem Punkt $M_f$ entspricht, ein Parameter $M_f$ eine Anpassungsmasse darstellt, ein Parameter $\lambda_f$ eine Anpassungslinienbreite darstellt und $\sigma_i$ ein bestimmter Parameter gemäß einer gegebenen Bedingung ist.

## Revendications

1.  Procédé de traitement numérique de spectre de masse d'acide nucléique, comprenant :

    étape S1 : recalibrage des spectres de masse, comprenant pour chaque point de détection d'un échantillon, l'obtention d'une pluralité de spectres de masse correspondant à différentes positions du point de détection, où chaque spectre de masse de la pluralité de spectres de masse est recalibré en utilisant un groupe de pics d'ancrage avec un rapport masse/charge attendu ;
    étape S2 : synthèse de la pluralité de spectres de masse, dans laquelle, sur la base de l'étape S1, la pluralité de spectres de masse correspondant aux différentes positions du point de détection est synthétisée en un spectre de masse unitaire du point de détection ;
    étape S3 : filtrage wavelet du spectre de masse unitaire du point de détection, sur la base de l'étape S2, afin d'éliminer le bruit à haute fréquence et la ligne de base au moyen d'un filtre numérique basé sur le wavelet ; et
    étape S4 : extraction d'une valeur caractéristique de pic, ajustement de pic pour obtenir une courbe ajustée du spectre de masse unitaire du point de détection sur la base de l'étape S3, et obtention d'une hauteur de pic, d'une largeur de pic, d'une zone de pic, d'un décalage de masse et d'un rapport signal-bruit sur la base de la courbe ajustée du spectre de masse unitaire du point de détection,

    dans lequel, à l'étape S1, le recalibrage de chaque spectre de masse unique comprend :

    étape S11 : sélection d'un groupe de pics de référence, la sélection d'un groupe de pics de référence comprenant la sélection du groupe de pics de référence parmi tous les pics attendus possibles en fonction des critères suivants :

    (1) une valeur de pic de référence se situe dans la plage de masse d'un intervalle spécifique, la plage de masse de l'intervalle spécifique étant comprise entre 4000 Da et 9000 Da;
    (2) le pic de référence n'a pas de pic de référence adjacent dans une plage de masse définie par la masse du pic de référence +/- la résolution ;

    étape S12 : positionnement d'un pic et application d'un filtre de convolution à matrice de poids d'une largeur de 9 au spectre de masse unique, le filtre de convolution à matrice de poids étant : (-4, 0, 1, 2, 2, 2, 1, 0, -4), pour un point donné du spectre de masse unique, une valeur d'intensité du point donné après application du filtre de convolution à matrice de poids est égale à une somme pondérée de 9 valeurs autour du point donné, et est exprimée par la formule suivante :

$$y_i' = \sum_{k=1}^{9} I_k y_k + i$$

    décomposer le spectre de masse unique en une pluralité d'intervalles de point spécifiques basés sur des valeurs d'intensité filtrées, identifier un bruit local pour chaque intervalle de point spécifique, et identifier un pic avec une intensité supérieure ou égale à quatre fois le bruit local et supérieure ou égale à une valeur minimale globale comme pic de référence, dans lequel la valeur minimale globale est : 0,01 * une valeur maximale locale ;

étape S13 : ajustement d'un pic du spectre de masse unique ;

étape S14 : sélection finale d'un pic d'ancrage pour une liste de pics détectés, détermination d'un rapport signal-bruit de coupure, mise en correspondance d'un pic de la liste de pics détectés avec une liste de pics de référence, et sélection d'un pic dont la masse se situe dans une plage spécifique du pic de référence et dont le rapport signal-bruit est supérieur au rapport signal-bruit de coupure ;

étape S15 : réalisation d'une opération recalibrage, calcul d'un coefficient d'étalonnage par un procédé d'ajustement non linéaire en combinaison avec le pic d'ancrage et une masse attendue du pic d'ancrage, une fonction de mise en correspondance entre un spectromètre de masse et le rapport masse/charge étant une fonction de Bruker sous la forme de $m = A(\sqrt{Bt + C} - 1)^2$ .

2. Procédé de traitement numérique de spectre de masse d'acide nucléique selon la revendication 1, dans lequel l'étape S2 comprend :

   synthétiser la pluralité de spectres de masse à l'aide d'un procédé de moyenne auto-pondérée,
   sélectionner un spectre de masse optimal avec les pics les plus ancrés parmi la pluralité de spectres de masse dans le cas où la pluralité de spectres de masse a des coefficients d'étalonnage différents ;
   initialiser un spectre de masse synthétisé en exécutant le procédé de moyenne auto-pondérée sur la pluralité de spectres de masse avec le spectre de masse optimal ;
   additionner une intensité absolue ou une intensité carrée d'un spectre de masse avec une intensité absolue ou une intensité carrée d'un autre spectre de masse uniquement dans le cas où un coefficient d'étalonnage du spectre de masse et un coefficient d'étalonnage du spectre optimal remplissent une condition, dans laquelle la condition est que A change à moins de 1 %, B change à moins de 10 % et C change à moins de 20 Da.

3. Procédé de traitement numérique de spectre de masse d'acide nucléique selon la revendication 1, dans lequel l'étape S3 comprend :
   effectuer un filtrage wavelet sur le spectre de masse unitaire du point de détection pour éliminer le bruit à haute fréquence et la ligne de base afin d'obtenir un spectre de masse filtré, puis effectuer une autre ronde de recalibrages sur le spectre de masse filtré et ajuster une valeur de rapport masse/charge en conséquence.

4. Procédé de traitement numérique de spectre de masse d'acide nucléique selon la revendication 1, dans lequel l'étape S4 comprend :

   étape S41 : ajustement d'un pic du spectre de masse unitaire pour obtenir un pic ajusté ;
   étape S42 : enregistrement des caractéristiques suivantes :

   (1) une hauteur au-dessus d'une ligne de base du centre du pic ajusté, $H_f$,
   (2) une largeur de ligne d'ajustement, $\lambda_f$,
   (3) un décalage de pic, qui est une distance entre le centre du pic ajusté et le centre d'un pic attendu, $\delta_f = M_f - M_e$,
   (4) une zone A d'une région entre le pic ajusté et la ligne de base dans une plage de $4\lambda_f$,
   (5) un rapport signal-bruit, $SNR = H_f/N(M)_f$,
   (6) une variance de surface, $V = A/SNR$,
   (7) une différence de surface d'ajustement $\Delta$, racine carrée de la somme de la différence carrée entre une intensité ajustée et une intensité mesurée.

5. Procédé de traitement numérique de spectre de masse d'acide nucléique selon la revendication 1, dans lequel à l'étape S13, les étapes d'ajustement d'un pic comprennent :

   étape S131 : détermination d'une largeur de ligne attendue ;
   étape S132 : masquage d'une région d'un signal attendu dans un intervalle de NN largeurs de lignes attendues, NN étant égal à 4 ;
   étape S133 : calcul d'une moyenne de l'intensité yi du spectre de masse unique dans un intervalle $MM\lambda m$ en tant que ligne de base implicite, où $\lambda m$ est la plus petite largeur de ligne estimée dans l'intervalle $MM\lambda m$, MM est égal à 80, et dans une région masquée de l'intervalle $MM\lambda m$, une valeur de l'intensité yi est fournie par interpolation linéaire ;

étape S134 : calcul d'une valeur effective d'un fonctionnement de la ligne de base-signal en tant que niveau de bruit ;

étape S135 : masquage d'un point, et masquage supplémentaire d'un point dont le rapport signal-bruit, calculé comme le rapport entre la hauteur du pic et le bruit, est supérieur à une valeur donnée pour le rapport et à une valeur donnée pour le bruit, dans une région de pic ;

étape S136 : détermination d'une région ayant des largeurs de lignes estimées spécifiques pour chaque pic en tant que région ajustée et, dans le cas où les pics ne se chevauchent pas, ajustement d'un pic gaussien unique par l'algorithme de Levenberg-Marquardt afin de trouver les paramètres spécifiés pour minimiser une fonction d'accord.

6. Procédé de traitement numérique de spectre de masse d'acide nucléique selon la revendication 2, dans lequel le procédé de moyenne auto-pondérée est décrit à l'aide d'une équation suivante :

$$\overline{I}_i = \frac{\sum_{j=1}^{n} I_{ij}^2}{\sum_{j=1}^{n} \left| I_{ij} \right|},$$

où n est le nombre de spectres de masse, $\overline{I}_i$ est l'intensité moyenne de la masse i ; $I_{ij}$ est l'intensité de la masse i du j$^{ème}$ spectre de masse.

7. Procédé de traitement numérique de spectre de masse d'acide nucléique selon la revendication 5, dans lequel à l'étape S 131, la largeur de ligne attendue déterminée est décrite par une équation suivante :

$$\lambda_e = L_A + L_B \cdot M$$

où $L_A$ et $L_B$ sont des paramètres par défaut, et M est une valeur de pic donnée.

8. Procédé de traitement numérique du spectre de masse d'acide nucléique selon la revendication 5, dans lequel la fonction d'accord à l'étape S136 est décrite par une équation suivante :

$$\chi^2 = \Sigma \left[ \frac{y_i - H_f \bullet \exp\left[ - (\frac{M_f - m_i}{\lambda_f})^2 \right]}{\sigma_i} \right]^2$$

une somme étant obtenue en additionnant tous les $\{y_i, m\}_i$ à partir d'un intervalle spécifié, $H_f$ est une hauteur d'ajustement au-dessus de la ligne de base correspondant à un point $M_f$, un paramètre $M_f$ représente une masse d'ajustement, un paramètre $\lambda_f$ représente une largeur de ligne d'ajustement, et $\sigma_i$ est un certain paramètre selon une condition donnée.

FIG. 1

FIG. 2

FIG. 3

**EP 4 016 379 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014306104 A1
- US 22016240359 A1
- EP 1469313 A1
- US 2003180748 A1